# DEMANDE DE BREVET EUROPEEN

(11) **EP 2 599 542 A1**
(43) Date de publication de la demande: **05.06.2013**
(21) Numéro de dépôt: 12290389.1
(22) Date de dépôt: 14.11.2012
(51) Int. Cl.: B01J 31/02

(54) **Composition catalytique et procédé pour la dimerisation sélective de l'isobutene**

(30) Priorité: 30.11.2011 FR 1103654
(71) Demandeur: IFP Energies Nouvelles, 92852 Rueil Malmaison (FR)
(72) Inventeur: Magna, Lionel, 69007 Lyon (FR); Olivier-Bourbigou, Helene, 69230 Saint Genis-Laval (FR)

(57) **Abrégé**

Composition catalytique comprenant au moins un acide de Brønsted, noté HB, dissous dans un milieu liquide non-aqueux de formule générale Q₁ ⁺A₁⁻, dans lequel Q₁⁺ représente un cation organique et A₁⁻ représente un anion, ladite composition comprenant en outre un additif Q₂⁺A₂⁻ dans lequel Q₂⁺ représente un cation organique contenant au moins une fonction alcool et A₂⁻ représente un anion. L'invention concerne également un procédé de dimérisation de l'isobutène utilisant la composition catalytique.

## Description

La présente invention concerne la dimérisation de l'isobutène. Plus particulièrement la présente invention a pour objet une nouvelle composition catalytique et un procédé de dimérisation de l'isobutène (pur ou en mélange avec d'autres hydrocarbures) utilisant cette nouvelle composition catalytique.

On sait que les dimères de l'isobutène (triméthyl-2,4,4-pentène-1 et -2) sont des intermédiaires intéressants pour la fabrication de différents produits ayant un intérêt commercial. A titre d'exemples, on peut citer les alcools supérieurs, les aldéhydes, les acides.

Le triméthyl-2,2,4-pentane peut être obtenu par hydrogénation des triméthylpentènes et constitue un additif recherché pour la reformulation des essences (absence de soufre, d'aromatique et d'oléfine, et faible volatilité s'ajoutent à un indice d'octane élevé : Indice d'Octane Moteur (RON) = Indice d'Octane Recherche (RON) = 100).

Ainsi, la dimérisation sélective de l'isobutène, suivie d'une hydrogénation des produits obtenus en triméthyl-2,2,4-pentane possédant un haut indice d'octane, constitue une voie intéressante qui permet
i) de remplacer le MTBE (Méthyl-Tert-Butyl-Ether : RON=118 ; MON=100), banni pour des raisons environnementales, et
ii) d'utiliser l'isobutène, issu des coupes C4 des procédés de craquage catalytique ou de craquage à la vapeur, matière première à la fabrication du MTBE.

La dimérisation (et aussi l'oligomérisation) de l'isobutène est une réaction exothermique catalysée par des acides. Différents acides ont été décrits dans la littérature tels que l'acide sulfurique, ou ses dérivés, les alumines chlorées ou fluorées, les zéolithes, les silices-alumines.... Cependant, les plus typiquement utilisés dans l'industrie sont l'acide phosphorique, généralement supporté, et les résines échangeuses d'ions (procédé SP-Isoéther licencié par Snamprogetti ou le procédé InAlk proposé par UOP).

La principale difficulté liée à ces procédés est l'obtention d'une bonne sélectivité en dimères. En effet, l'exothermicité de la réaction est souvent difficile à contrôler et entraîne la formation d'oligomères (essentiellement des oléfines en C12 et des oléfines en C16) obtenus par réactions parallèles à partir de l'isobutène. Ces oligomères ont des points d'ébullition trop élevés, et se trouvent en dehors, ou à la limite, des spécifications exigées pour les essences reformulées. Par ailleurs, ces oligomères contribuent à désactiver les catalyseurs.

Différents travaux dans la littérature décrivent certaines solutions pour minimiser la formation de ces oligomères.

Dans le cas des résines échangeuses d'ions (type Amberlyst-15 ou -35), l'utilisation d'un diluent (ou solvant) est souvent préconisée. La sélectivité en dimères dépend du choix de ce solvant. Les additifs les plus efficaces sont les alcools (US-A-5 877 372 ; US-A-4 100 220), qui conduisent à la co-production d'éthers, ou les éthers (dans US-A-4 447 668, le MTBE, l'ETBE, etc.). On peut citer les travaux de Snamprogetti (M. Marchionna and al. Catal. Today, 65 (2001) 397-403, GB 2 325 237) qui ont étudié l'influence de l'addition de MTBE ou de MeOH dans l'objectif de réutiliser les unités existantes de MTBE. Des sélectivités intéressantes en triméthylpentènes peuvent ainsi être atteintes mais à des conversions de l'isobutène souvent inférieures à 85 %.

La demande de brevet internationale WO-A-01/51 435 décrit un enchaînement de procédés dans lequel l'isobutène est produit par déhydratation de l'alcool tert-butylique. L'isobutène est dimérisé préférentiellement par une résine type Amberlyst A-15® en présence d'alcool tert-butylique (promoteur de sélectivité) et d'alcane (butane ou isobutane) comme diluant. La présence d'alcool encombré défavorise la formation d'éther mais aussi diminue la vitesse de réaction.

Tous les procédés décrits précédemment possèdent des limitations telles que les risques de désactivation prématurée du catalyseur par « encrassement » par les oligomères plus lourds ou encore la nécessité d'utiliser des additifs organiques pour maitriser la sélectivité de la réaction, additifs organiques souvent transformés et consommés lors de la réaction et donc non recyclables.

Les liquides ioniques non-aqueux de composition Q⁺A⁻ ont fait l'objet de plusieurs revues (par exemple, H. Olivier-Bourbigou et al., Appl. Catal. A: General, 2010, 1-56). Ils trouvent de nombreuses applications comme solvants pour la catalyse par les métaux de transition ou comme solvants d'extraction pour réaliser des extractions liquide-liquide.

La demande de brevet WO-A-00/16902 décrit l'utilisation d'un liquide ionique ne contenant pas d'acidité de Lewis obtenu par réaction d'un composé azoté (par exemple une amine ou un ammonium quaternaire) ou phosphoré avec un acide de Bronsted en quantités telles que le rapport dudit composé azoté ou phosphoré sur l'acide soit inférieur à 1. Ces milieux sont utilisés pour catalyser notamment l'alkylation du benzène avec le décène-1.

Il a également été décrit dans la demande FR2829039 que l'addition d'au moins un acide de Brønsted, noté HB, dans un milieu liquide non-aqueux (milieu de type « sel fondu ») comprenant au moins un cation organique Q⁺ et un anion A⁻ et dans laquelle, lorsque A et B sont identiques, le rapport molaire de l'acide de Brønsted sur le liquide ionique est inférieur à 1/1, conduit à des compositions liquides qui peuvent être utilisées comme catalyseurs et solvants pour des réactions de catalyse acide.

Dans cette demande de brevet, il était mentionné que la composition catalytique décrite pouvait être utilisée plus particulièrement dans l'alkylation d'hydrocarbures aromatiques, mais aussi dans l'oligomérisation des oléfines, la dimérisation de l'isobutène, l'alkylation de l'isobutane par les oléfines, l'isomérisation des n-paraffines en iso-paraffines et l'isomérisation des n-oléfines en iso-oléfines.

L'utilisation des liquides ioniques comme solvants et catalyseurs acides pour la dimérisation sélective de l'isobutène a aussi été décrite dans le brevet US7256152. L'avantage de ces systèmes catalytiques liquides pour la réaction de dimérisation de l'isobutène en isooctènes est qu'ils sont peu miscibles avec les produits de la réaction, ceux-ci pouvant ainsi être séparés par décantation. La phase catalytique peut alors être recyclée et réutilisée, les consommations de catalyseur sont ainsi réduites. Cependant, ces systèmes possèdent encore des limitations. A l'instar des catalyseurs hétérogènes, il est parfois difficile de maintenir une bonne sélectivité en produits de dimérisation à haute conversion d'isobutène.

L'objet de la présente invention est de fournir une nouvelle composition catalytique permettant d'améliorer la sélectivité de la réaction de dimérisation de l'isobutène.

En plus, la composition catalytique peut être recyclée sans baisse notable de son activité catalytique et sans baisse notable de sa sélectivité en dimères. L'additif compris dans la composition catalytique selon l'invention n'est donc ni transformé ni consommé lors de la réaction et est donc recyclable. Il n'est donc pas nécessaire d'injecter un additif en continu.

Plus particulièrement, la présente invention concerne une composition catalytique comprenant au moins un acide de Brønsted, noté HB, dissous dans un milieu liquide non-aqueux de formule générale Q₁⁺A₁⁻, dans lequel Q₁⁺ représente un cation organique et A₁⁻ représente un anion, ladite composition comprenant en outre un additif Q₂⁺A₂⁻ dans lequel Q₂⁺ représente un cation organique comprenant au moins une fonction alcool et A₂⁻ représente un anion.

Le liquide ionique Q₁⁺A₁⁻, dans lequel est dissous l'acide de Brønsted HB, est défini tel que Q₁⁺ représente un ammonium quaternaire et/ou un phosphonium quaternaire et/ou un trialkylsulfonium et A₁⁻ représente tout anion connu comme étant non-coordinant susceptible de former un sel liquide à basse température, c'est-à-dire en dessous de 150°C. L'additif Q₂⁺A₂⁻ est défini tel que Q₂⁺ représente un ammonium quaternaire et/ou un phosphonium quaternaire et/ou un trialkylsulfonium comprenant au moins une fonction alcool et A₂⁻ représente tout anion connu comme étant non-coordinant susceptible de former un sel liquide à basse température, c'est-à-dire en dessous de 150°C.

Les anions A₁⁻ ou A₂⁻ considérés dans l'invention seront de préférence choisis parmi les anions tétrafluoroborate, tétraalkylborates, hexafluorophosphate, hexafluoroantimonate, les alkylsulfonates et les arylsulfonates (par exemple le méthylsulfonate ou le tosylate), les perfluoroalkylsulfonates (par exemple le trifluorométhylsulfonate), le fluorosulfonate, les sulfates, les phosphates, les perfluoroacétates (par exemple le trifluoroacétate), les perfluoroalkylsulfonamides (par exemple l'amidure de bis-trifluorométhanesulfonyle (N(CF₃SO₂)₂⁻), les fluorosulfonamides, les perfluoroalkylsulfométhides (par exemple le méthylure de tris-trifluorométhanesulfonyle (C(CF₃SO₂)₃⁻) et les carboranes, A₁⁻ et A₂⁻ étant identiques ou différents. De préférence, A₁⁻ et A₂⁻ sont identiques.

Les cations Q₁⁺ et Q₂⁺ considérés dans l'invention seront de préférence choisis parmi les ammoniums quaternaires et/ou les phosphoniums quaternaires et/ou les trialkylsulfoniums. La nature chimique de Q₁⁺ et Q₂⁺ peut être identique ou différente, elle est de préférence identique. On entend par nature chimique identique que si, par exemple, le cation Q₁⁺ est un ammonium quaternaire, Q₂⁺ est également un ammonium quaternaire. On entend par nature chimique différente que si, par exemple, le cation Q₁⁺ est un ammonium quaternaire, Q₂⁺ est choisi parmi un phosphonium quaternaire ou un trialkylsulfonium. Dans tout les cas, le cation organique Q₂⁺ de l'additif Q₂⁺A₂⁻ contient au moins une fonction alcool, alors que les substituants du cation organique Q₁⁺ n'ont pas de fonction alcool. On entend par fonction alcool un groupement OH greffé sur le cation Q₂⁺, soit directement sur un carbone du cation Q₂⁺, soit par l'intermédiaire d'un groupement aryle, aralkyle, alkyle ou cycloalkyle, comprenant de préférence de 1 à 12 atomes de carbone.

Les ammoniums et/ou phosphoniums quaternaires de formule générale Q₁⁺ et Q₂⁺ répondent de préférence aux formules NR¹R²R³R⁴⁺ et PR¹R²R³R⁴⁺, ou aux formules générales R¹R²N=CR³R⁴⁺ et R¹R²P=CR³R⁴⁺ où
- pour Q₁⁺ : R¹, R², R³ et R⁴, identiques ou différents, représentent l'hydrogène, à l'exception du cation NH₄⁺ pour NR¹R²R³R⁴⁺, et de préférence un seul substituant peut représenter l'atome d'hydrogène, ou des restes hydrocarbyles ayant de 1 à 12 atomes de carbone, par exemple des groupements alkyles, saturés ou non saturés, cycloalkyles ou aromatiques, aryle ou aralkyle, comprenant de 1 à 12 atomes de carbone,
- et pour Q₂⁺: au moins un substituent R¹, R², R³ ou R⁴ contient au moins une fonction alcool étant définie comme un groupement OH greffé sur le cation Q₂⁺, soit directement sur un carbone du cation Q₂⁺, soit par l'intermédiaire d'un groupement aryle, aralkyle, alkyle ou cycloalkyle, comprenant de préférence de 1 à 12 atomes de carbone, les substituants n'ayant pas de fonction alcool, identiques ou différents, sont définis comme précédemment pour Q₁⁺,

Les ammoniums et/ou phosphoniums quaternaires de formule générale Q₁⁺ et Q₂⁺ peuvent également être dérivés d'hétérocycles azotés (imidazolium, pyridinium, pyrrolidinium, pyrazolium, triazolium) ou phosphorés comportant 1, 2 ou 3 atomes d'azote et/ou de phosphore, de formules générales : 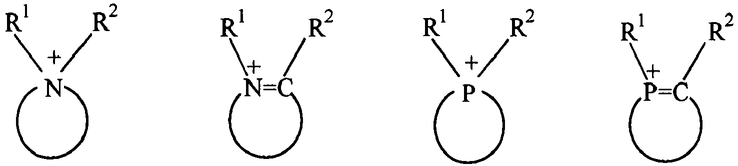 dans lesquelles les cycles sont constitués de 4 à 10 atomes, de préférence 5 à 6 atomes, R¹ et R² pour Q₁⁺ ou Q₂⁺ étant définis comme précédemment.

Les ammoniums et/ou phosphoniums quaternaires de formule générale Q₁⁺ et Q₂⁺ peuvent également consister en un cation répondant à l'une des formules générales :
R¹R²+N=CR³-R⁵-R³C=N⁺R¹R² et
R¹R²⁺P=CR³-R⁵-R³C=P⁺R¹R²
dans lesquelles R¹, R² et R³, identiques ou différents, sont définis comme précédemment pour Q₁⁺ ou Q₂⁺ et R⁵ représente un reste alkylène ou phénylène.

Parmi les groupements R¹, R², R³ et R⁴ on mentionnera les radicaux méthyle, éthyle, propyle, isopropyle, butyle, butyle secondaire, butyle tertiaire, amyle, phényle ou benzyle ; R⁵ pourra être un groupement méthylène, éthylène, propylène ou phénylène.

Parmi les groupements R¹, R², R³ et R⁴ contenant une fonction alcool, on mentionnera les radicaux -CH₂OH, -(CH₂)₂OH, -(CH₂)₃OH, -(CH₂)₄OH, -C(CH₃)₂-CH₂OH, -CH₂-C(CH₃)₂OH, -C(CH₃)H-CH₂OH, -C₆H₄-CH₂OH.

Le cation ammonium et/ou phosphonium quaternaire Q₁⁺ est choisi de préférence dans le groupe formé par le N-butylpyridinium, le N-éthylpyridinium, le 1-butyl-3-méthylimidazolium, le diéthylpyrazolium, le 1-éthyl-3-méthylimidazolium, le pyridinium, le triméthylphénylammonium, le tétrabutylphosphonium, le N-éthyl-N-méthylpyrrolidinium et le N-butyl-N-éthylpyrrolidinium.

Le cation ammonium et/ou phosphonium quaternaire Q₂⁺ est choisi de préférence dans le groupe formé par le 1-butyl-3-(2-hydroxyéthyl)imidazolium, 1-éthyl-3-(2-hydroxyéthyl)imidazolium, N-butyl-N-(2-hydroxyéthyl)pyrrolidinium, N-éthyl-N-(2-hydroxyéthyl)pyrrolidinium, (2-hydroxyéthyl)triéthylammonium et le triphényl(3-hydroxypropyl)phosphonium.

Les trialkylsulfoniums Q₁⁺ ou Q₂⁺ considérés dans l'invention ont pour formule générale SR¹R²R³⁺ dans laquelle
- pour Q₁⁺ : R¹, R² et R³ identiques ou différents, représentent des restes hydrocarbyles ayant de 1 à 12 atomes de carbone, par exemple des groupements alkyles ou alkényles, cycloalkyles ou aromatiques, aryles ou aralkyles, comprenant de 1 à 12 atomes de carbone,
- et pour Q₂⁺ : au moins un substituent R¹, R² ou R³ représente une fonction alcool étant définie comme un groupement OH greffé sur le cation Q₂⁺, soit directement sur un carbone du cation Q₂⁺, soit par l'intermédiaire d'un groupement aryle, aralkyle, alkyle ou cycloalkyle, comprenant de préférence de 1 à 12 atomes de carbone, les substituants n'ayant pas de fonction alcool, identiques ou différents, sont définis comme précédemment pour Q₁⁺.

A titre d'exemples des liquides ioniques utilisables Q₁⁺A₁⁻, on peut citer l'hexafluorophosphate de N-butylpyridinium, le tétrafluoroborate de N-éthyl pyridinium, l'hexafluoroantimonate de 1-butyl-3-méthylimidazolium, l'hexafluorophosphate de 1-butyl-3-méthylimidazolium, le trifluorométhylsulfonate de 1-butyl-3-méthylimidazolium, le fluorosulfonate de pyridinium, l'hexafluorophosphate de triméthylphénylammonium, le bis-trifluorométhylsulfonylamidure de 1-butyl-3-méthylimidazolium, le bis-trifluorométhylsulfonylamidure de N-éthyl-N-méthylpyrrolidinium, le bis-trifluorométhylsulfonylamidure de triéthylsulfonium, le bis-trifluorométhylsulfonylamidure de tributylhexylammonium, le trifluoroacétate de 1-butyl-3-méthylimidazolium et le bis-trifluorométhylsulfonylamidure de 1-butyl-2,3-diméthylimidazolium.

Ces liquides ioniques peuvent être utilisés seuls ou en mélange. Ils ont une fonction de solvants.

A titre d'exemples des additifs Q₂⁺A₂⁻ utilisables, on peut citer le l'hexafluorophosphate de N-(4-hydroxybutyl)pyridinium, le tétrafluoroborate de N-(2-hydroxyéthyl)pyridinium, l'hexafluoroantimonate de 1-(4-hydroxybutyl)-3-méthylimidazolium, l'hexafluorophosphate de 1-(4-hydroxybutyl)-3-méthylimidazolium, le trifluorométhylsulfonate de 1-(4-hydroxybutyl)-3-méthylimidazolium, le fluorosulfonate de N-(2-hydroxyethyl)pyridinium, l'hexafluorophosphate de triméthyl(2-hydroxyethyl)ammonium, le bis-trifluorométhylsulfonylamidure de 1-(4-hydroxybutyl)-3-méthylimidazolium, le bis-trifluorométhylsulfonylamidure de N-(2-hydroxyethyl)-N-méthylpyrrolidinium, le bis-trifluorométhylsulfonylamidure de diéthyl(2-hydroxyéthyl)sulfonium, le bis-trifluorométhylsulfonylamidure de tributyl(4-hydroxybutyl)ammonium, le trifluoroacétate de 1-(4-hydroxybutyl)-3-méthylimidazolium, le bis-trifluorométhylsulfonylamidure de 1-(4-hydroxybutyl)-2,3-diméthylimidazolium et le le bis-trifluorométhylsulfonylamidure de 1-butyl-3-(2-hydroxyéthyl)imidazolium.

Ces additifs peuvent être utilisés seuls ou en mélange. Ils permettent d'améliorer la sélectivité en dimères et sont recyclables.

De façon préférée, dans la composition catalytique utilisée selon l'invention, les anions A₁⁻ et A₂⁻ sont identiques. Le rapport molaire entre l'additif Q₂⁺A₂⁻ et le liquide ionique Q₁⁺A₁⁻ est de préférence inférieur à 2/1, et de manière encore plus préférée inférieur à 1/1.

Les acides de Brønsted sont définis comme étant des composés organiques acides susceptibles de donner au moins un proton. Ces acides de Brønsted ont pour formule générale HB, dans laquelle B représente un anion.

Les anions B sont de préférence choisis parmi les anions tétrafluoroborate, tétraalkylborates, hexafluorophosphate, hexafluoroantimonate, les alkylsulfonates et les arylsulfonates (par exemple le méthylsulfonate ou le tosylate), les perfluoroalkylsulfonates (par exemple le trifluorométhylsulfonate), le fluorosulfonate, les sulfates, les phosphates, les perfluoroacétates (par exemple le trifluoroacétate), les perfluoroalkylsulfonamides (par exemple l'amidure de bis-trifluorométhanesulfonyl (N(CF₃SO₂)₂⁻), les fluorosulfonamides, les perfluoroalkylsulfométhides (par exemple le méthylure de tris-trifluorométhanesulfonyle (C(CF₃SO₂)₃⁻) et les carboranes.

Les acides de Brønsted peuvent être utilisés seuls ou en mélange. De façon préférée, dans la formule des acides de Brønsted utilisés, les anions B, A₁⁻ et A₂⁻ sont identiques.

Dans tous les cas, le rapport molaire de l'acide de Brønsted sur la somme de (Q₁⁺A₁⁻ + Q₂⁺A₂⁻) est inférieur à 2/1, de préférence inférieur à 1/1.

Les composés entrant dans la composition catalytique utilisée dans le procédé de l'invention peuvent être mélangés dans un ordre quelconque. Le mélange peut se faire par une simple mise en contact suivie d'une agitation jusqu'à formation d'un liquide homogène. Ce mélange peut être fait en dehors du réacteur utilisé pour l'application catalytique ou dans ce réacteur.

La présente invention a également comme objet un procédé de dimérisation d'isobutène mettant en jeu la composition catalytique. Le procédé de dimérisation selon l'invention s'applique à l'isobutène pur ou en mélange avec d'autres hydrocarbures.

Les provenances de l'isobutène sont diverses. Cependant, les plus courantes sont la déshydrogénation de l'isobutane, la déshydratation de l'alcool tert-butylique. L'isobutène peut aussi provenir d'une coupe C4 d'un craquage catalytique en lit fluidisé ou d'un craquage à la vapeur. Dans ce dernier cas, l'isobutène peut être utilisé en mélange avec les n-butènes, l'isobutane et le butane. Le procédé selon l'invention présente alors l'avantage supplémentaire de permettre de convertir sélectivement l'isobutène sans qu'on ait à le séparer des autres constituants de la coupe. Un autre avantage du procédé selon l'invention est que la co-dimérisation isobutène-butène peut être limitée.

Des avancées récentes dans le domaine des biotechnologies montrent qu'il est également possible de générer de l'isobutène à partir du 2-méthyl-propanol (isobutanol), lui même obtenu à partir de sucres issues de la fermentation de la biomasse.

Le rapport volumique entre l'isobutène et la composition catalytique peut être compris entre 0,1/1 et 1000/1, de préférence entre 1/1 et 100/1. Il sera choisi de façon à obtenir les meilleures sélectivités.

La réaction peut être conduite en système fermé, en système semi-ouvert ou en continu avec un ou plusieurs étages de réaction. A la sortie du réacteur, la phase organique contenant les produits de réaction est séparée.

Dans le procédé de dimérisation de l'invention, on peut ajouter à la composition catalytique un solvant organique comme un hydrocarbure aliphatique ou un hydrocarbure aromatique non ou partiellement miscible avec le liquide ionique qui permet une meilleure séparation des phases. On peut utiliser comme hydrocarbure aliphatique par exemple le pentane, l'heptane, le cyclohexane, le décane, le dodécane ou une charge paraffinique, seul ou en mélange. On peut utiliser comme hydrocarbure aromatique par exemple le toluène ou le xylène, seul ou en mélange.

La température à laquelle on effectue la réaction de dimérisation va par exemple de -50°C à 200°C ; elle est avantageusement inférieure à 100°C.

La réaction peut se faire à la pression autogène, celle-ci peut aussi être augmentée jusqu'à 10 MPa.

La réaction de dimérisation peut être conduite en utilisant une technique de distillation réactive.

Les produits obtenus par la présente invention peuvent être transformés ultérieurement selon différentes réactions, telles que hydrogénation, hydroformylation, oxydation, éthérification, époxydation ou hydratation.

Les exemples suivants illustrent l'invention sans en limiter la portée.

### Exemple 1 (selon l'invention) : Préparation de l'additif bis(trifluoromethylsulfonyl)amidure de 1-butyl-3-(2-hydroxyéthyl)imidazolium [BuIm(CH₂)₂OH][N(CF₃SO₂)₂] noté "(Q₂⁺A₂⁻)" selon l'invention :

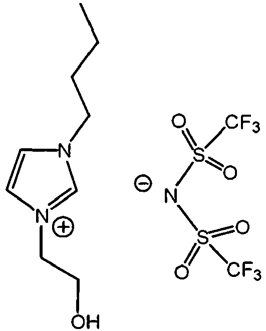

Dans un ballon de 500 ml on introduit sous atmosphère inerte 20 g (0,16 mol) de N-butylimidazole ainsi que 25,9 g (0,32 mol) de 1-chloroéthanol. On additionne ensuite 150 mL d'acétonitrile. Le mélange est porté au reflux durant 5 jours. Après retour à température, l'acétonitrile est éliminée sous vide. On obtient un liquide visqueux de couleur jaune. Ce composé est dissout dans 100 mL de dichlorométhane. On additionne ensuite 48,2 g (0,168mol) de bis(trifluorométhylsulfonyl)amidure de lithium. Après 2h de réaction à température ambiante, le mélange est filtré sur "célite 545/Al₂O₃ neutre" avant d'évaporer le solvant sous vide dynamique. Le résidu liquide est lavé plusieurs fois avec de l'eau puis séché à 80°C sous vide dynamique. On obtient 34,5 g (rendement 48%) de [BuIm(CH₂)₂OH][N(CF₃SO₂)₂]. Les analyses RMN ¹H et ¹³C confirme la structure du produit attendu.

### Exemple 2 (selon l'invention) : Préparation du système catalytique "[BuIm(CH₂)₂OH][N(CF₃SO₂)₂] / [BMIm][N(CF₃SO₂)₂] HN(CF₃SO₂)₂" noté "Q₂⁺A₂⁻/ Q₁⁺A₁⁻ / HB" selon l'invention :

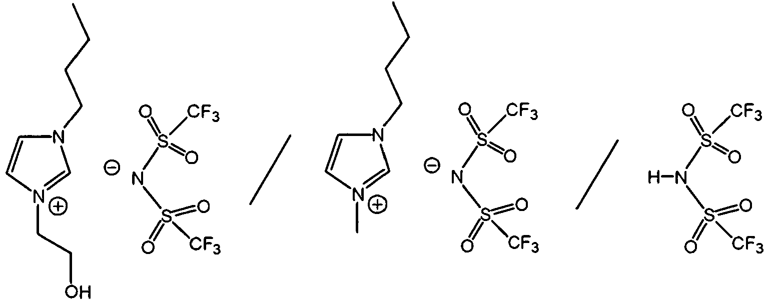

On a mélangé à température ambiante, sous atmosphère inerte, 4,97 g (11,9 mmol) de bis(trifluoromethylsulfonyl)amidure de 1-butyl-3-méthylimidazolium [BMIm][N(CF₃SO₂)₂)], avec 0,85g (1,9 mmol) de [BuIm(CH₂)₂OH][N(CF₃SO₂)₂] décrit dans l'exemple 1. L'ensemble représente un volume de 4 mL. On additionne ensuite 0,014 g (0,051 mmoles) d'acide bis-triflylamidure HN(CF₃SO₂)₂. Le mélange est agité quelques minutes et conduit à une solution limpide contenant 0,013 mol/L d'acide.

### Exemple 3 (contre exemple) : Préparation du système catalytique [BMIm][N(CF₃SO₂)₂] / HN(CF₃SO₂)₂ noté "Q₁⁺A₁⁻ / HB"

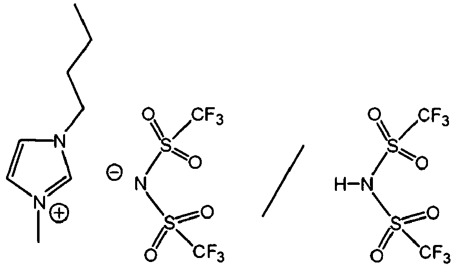

On a mélangé à température ambiante, sous atmosphère inerte, 4 mL (5,5 g, 13,1 mmol) de bis(trifluorométhylsulfonyl)amidure de 1-butyl-3-méthylimidazolium [BMIm][N(CF₃SO₂)₂), avec 0,015 g (0,054 mmoles) d'acide bis-triflylamidure HN(CF₃SO₂)₂. Le mélange est agité quelques minutes et conduit à une solution limpide contenant 0,013 mol/L d'acide.

### Exemple 4 (contre exemple) : Préparation du système catalytique [BuIm(CH₂)₂OH][N(CF₃SO₂)₂] / HN(CF₃SO₂)₂ noté "Q₂⁺A₂⁻ / HB"

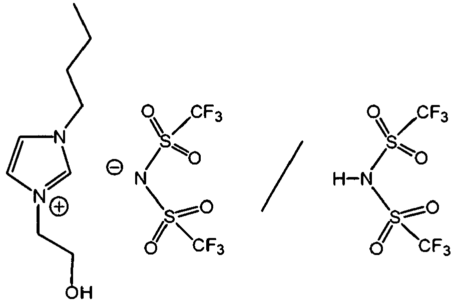

On a mélangé à température ambiante, sous atmosphère inerte, 4 mL (4,8 g, 10,7 mmol) de [BuIm(CH₂)₂OH][N(CF₃SO₂)₂] décrit dans l'exemple 1, avec 0,014 g (0,013 mmoles) d'acide bis-triflylamidure HN(CF₃SO₂)₂. Le mélange est agité quelques minutes et conduit à une solution limpide contenant 0,013 mol/L d'acide.

### Exemple 5 (selon l'invention) : Dimérisation de l'isobutène utilisant la composition catalytique de l'Exemple 2

Dans un tube Fisher-Porter de volume 100 mL, muni d'un barreau magnétique et préalablement séché à l'étude et tiré sous vide, on introduit, sous atmosphère d'argon, la totalité du mélange préparé dans l'Exemple 2. Puis, on introduit, à température ambiante, 20 mL (11,2g) d'une charge liquide contenant 95 % d'isobutène et 5 % de n-butane. L'agitation est alors mise en route (temps zéro de la réaction). Après 20 minutes de réaction à 25°C, l'agitation est arrêtée. La phase organique surnageante est séparée de la phase liquide ionique et analysée par CPV (chromatographie en phase vapeur, avec l'heptane comme étalon externe) après traitement à la soude (10N) pour éliminer les éventuelles traces d'acide et séchage sur MgSO₄. La conversion de l'isobutène est de 73%. La sélectivité en produit de dimérisation (C8) est de 74%, la sélectivité en trimères (C12) est de 23 %, celle en tetramères (C16) de 3%.

La phase liquide ionique contenant le mélange "[BuIm(CH₂)₂OH][N(CF₃SO₂)₂] / [BMIm][N(CF₃SO₂)₂] / HN(CF₃SO₂)₂ a été isolée et réutilisée sur plusieurs cycles de catalyse sans ajustement de la composition catalytique (exemples 6 à 11). Les résultats obtenus sont reportés dans le tableau 1.

### Exemple 12 (contre exemple) : Dimérisation de l'isobutène utilisant la composition catalytique de l'Exemple 3

Le protocole est identique à celui décrit dans l'exemple 5 à ce ci près que l'on utilise la composition catalytique décrite dans l'exemple 3. Après 7 minutes de réaction à 25°C, l'agitation est arrêtée. La phase organique surnageante est séparée de la phase liquide ionique et analysée par CPV. La conversion de l'isobutène est de 84%. La sélectivité en produit de dimérisation (C8) est de 47%, la sélectivité en trimères (C12) est de 46 %, celle en tetramères (C16) de 6% (voir tableau 2).

### Exemple 13 (contre exemple) : Dimérisation de l'isobutène utilisant la composition catalytique de l'Exemple 4

Le protocole est identique à celui décrit dans l'exemple 5 à ce ci près que l'on utilise la composition catalytique décrite dans l'exemple 4. Après 20 minutes de réaction à 25°C, l'agitation est arrêtée. La phase organique surnageante est séparée de la phase liquide ionique et analysée par CPV. Aucune conversion de l'isobutène n'a été observée (voir tableau 2).

**Tableau 1 :**

| Ex | Q₁⁺A₁⁻ | Q₂⁺A₂⁻ | HB | Conv. iC₄⁼ | Sélectivités (%pds) | | | |
|---|---|---|---|---|---|---|---|---|
| | | | | | C8 | C12 | C16 | C16+ |
| 5 | [BMIm] [N(CF₃SO₂)₂] | [BuIm(CH₂)₂O H] [N(CF₃SO₂)₂] | HN(CF₃SO₂)₂ | 73 | 74 | 23 | 3 | < 0.5 |
| 6 | Recyclage | | | 75 | 71 | 26 | 3 | < 0.5 |
| 7 | Recyclage | | | 79 | 71 | 26 | 3 | < 0.5 |
| 8 | Recyclage | | | 79 | 71 | 26 | 3 | < 0.5 |
| 9 | Recyclage | | | 67 | 74 | 23 | 3 | < 0.5 |
| 10 | Recyclage | | | 72 | 73 | 23 | 4 | < 0.5 |
| 11 | Recyclage | | | 73 | 74 | 23 | 3 | < 0.5 |

**Tableau 2 :**

| E x | Q₁⁺A₁⁻ | Q₂⁺A₂⁻ | HB | Conv. iC₄⁼ | Sélectivités (%pds) | | | |
|---|---|---|---|---|---|---|---|---|
| | | | | | C8 | C12 | C16 | C16+ |
| 12 | [BMIm] [N(CF₃SO₂)₂] | - | HN(CF₃SO₂)₂ | 84 | 47 | 26 | 6 | <0,5 |
| 13 | - | [BuIm(CH₂)₂O H] [N(CF₃SO₂)₂] | HN(CF₃SO₂)₂ | - | - | - | - | < 0.5 |

La composition catalytique contenant l'additif Q₂⁺A₂⁻ selon l'invention permet d'obtenir une meilleure sélectivité en dimères C8 par rapport à une composition catalytique sans additif. On voit également que le système catalytique est recyclable.

## Revendications

1. Composition catalytique comprenant au moins un acide de Brønsted, noté HB, dissous dans un milieu liquide non-aqueux de formule générale Q₁⁺A₁⁻, dans lequel Q₁⁺ représente un cation organique et A₁⁻ représente un anion, ladite composition comprenant entre outre un additif Q₂⁺A₂⁻ dans lequel Q₂⁺ représente un cation organique comprenant au moins une fonction alcool et A₂⁻ représente un anion.

2. Composition catalytique selon la revendication 1, dans laquelle l'anion A₁⁻ ou A₂⁻ est choisi parmi les anions tétrafluoroborate, tétraalkylborates, hexafluorophosphate, hexafluoroantimonate, alkylsulfonates, arylsulfonates, perfluoroalkylsulfonates, fluorosulfonate, sulfates, phosphates, perfluoroacétates, perfluoroalkylsulfonamides, fluorosulfonamides, perfluoroalkylsulfométhides et carboranes, A₁⁻ et A₂⁻ étant identiques ou différents.

3. Composition catalytique selon la revendication 1 ou 2, dans laquelle Q₁⁺ représente un ammonium quaternaire et/ou un phosphonium quaternaire + et/ou un trialkylsulfonium, et Q₂⁺ représente un ammonium quaternaire et/ou un phosphonium quaternaire et/ou un trialkylsulfonium comprenant au moins une fonction alcool, et A₁⁻ ou A₂⁻ représentent tout anion connu comme étant non-coordinant et susceptible de former un sel liquide au-dessous de 150°C.

4. Composition catalytique selon la revendication 3, dans laquelle le cation ammonium et/ou phosphonium quaternaire Q₁⁺ ou Q₂⁺ est choisi parmi :
- les cations ammonium et/ou phosphonium quaternaires répondant à l'une des formules générales NR¹R²R³R⁴⁺ et PR¹R²R³R⁴⁺, ou à l'une des formules générales R¹R²N=CR³R⁴⁺ et R¹R²P=CR³R⁴⁺ où,
- pour Q₁⁺ : R¹, R², R³ et R⁴, identiques ou différents, représentent l'hydrogène, à l'exception du cation NH₄⁺ pour NR¹R²R³R⁴⁺, un seul substituant représentant l'atome d'hydrogène, ou des restes hydrocarbyles ayant de 1 à 12 atomes de carbone,
- et pour Q₂⁺ : au moins un substituent R¹, R², R³ ou R⁴ contient au moins une fonction alcool étant définie comme un groupement OH greffé sur le cation Q₂⁺, soit directement sur un carbone du cation Q₂⁺, soit par l'intermédiaire d'un groupement aryle, aralkyle, alkyle ou cycloalkyle, comprenant de préférence de 1 à 12 atomes de carbone, les substituants n'ayant pas de fonction alcool, identiques ou différents, sont définis comme précédemment pour Q₁⁺,
- les cations ammonium et/ou phosphonium quaternaires dérivés d'hétérocycles azotés ou phosphorés comportant 1, 2 ou 3 atomes d'azote et/ou de phosphore, de formules générales : 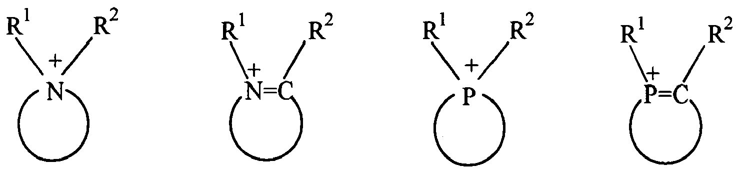 dans lesquelles les cycles sont constitués de 4 à 10 atomes, de préférence 5 à 6 atomes, R¹ et R² pour Q₁⁺ ou Q₂⁺ étant définis comme précédemment,
- les cations ammonium et/ou phosphonium quaternaires répondant à l'une des formules générales :
R¹R2⁺N=CR³-R⁵-R³C-N⁺R¹R²
R¹R²⁺P=CR³-R⁵-R³C=P⁺R¹R²
dans lesquelles R¹, R² et R³, identiques ou différents, sont définis comme précédemment pour Q₁⁺ ou Q₂⁺ et R⁵ représente un reste alkylène ou phénylène.

5. Composition catalytique selon la revendication 4, dans laquelle le cation ammonium et/ou phosphonium quaternaire Q₁⁺ est choisi dans le groupe formé par le N-butylpyridinium, le N-éthylpyridinium, le 1-butyl-3-méthylimidazolium, le diéthylpyrazolium, le 1-éthyl-3-méthylimidazolium, le pyridinium, le triméthylphénylammonium, le tétrabutylphosphonium, le N-éthyl-N-méthylpyrrolidinium et le N-butyl-N-éthylpyrrolidinium.

6. Composition catalytique selon la revendication 4, dans laquelle le cation ammonium et/ou phosphonium quaternaire Q₂⁺ est choisi dans le groupe formé par le 1-butyl-3-(2-hydroxyéthyl)imidazolium, 1-éthyl-3-(2-hydroxyéthyl)imidazolium, N-butyl-N-(2-hydroxyéthyl)pyrrolidinium, N-éthyl-N-(2-hydroxyéthyl)pyrrolidinium, (2-hydroxyéthyl)triéthylammonium et le triphényl(3-hydroxypropyl)phosphonium.

7. Composition catalytique selon la revendication 3, dans laquelle Q₁⁺ ou Q₂⁺ est un cation trialkylsulfonium répondant à la formule générale SR¹R²R³⁺ dans laquelle
- pour Q₁⁺: R¹, R² et R³, identiques ou différents, représentent des restes hydrocarbyles ayant de 1 à 12 atomes de carbone,
- et pour Q₂⁺ : au moins un substituent R¹, R² ou R³ représente une fonction alcool étant définie comme un groupement OH greffé sur le cation Q₂⁺, soit directement sur un carbone du cation Q₂⁺, soit par l'intermédiaire d'un groupement aryle, aralkyle, alkyle ou cycloalkyle, comprenant de préférence de 1 à 12 atomes de carbone, les substituants n'ayant pas de fonction alcool, identiques ou différents, sont définis comme précédemment pour Q₁⁺.

8. Composition catalytique selon l'une des revendications précédentes, dans laquelle le liquide ionique Q₁⁺A₁⁻ est choisi parmi l'hexafluorophosphate de N-butylpyridinium, le tétrafluoroborate de N-éthyl pyridinium, l'hexafluoroantimonate de 1-butyl-3-méthylimidazolium, l'hexafluorophosphate de 1-butyl-3-méthylimidazolium, le trifluorométhylsulfonate de 1-butyl-3-méthylimidazolium, le fluorosulfonate de pyridinium, l'hexafluorophosphate de triméthylphénylammonium, le bis-trifluorométhylsulfonylamidure de 1-butyl-3-méthylimidazolium, le bis-trifluorométhylsulfonylamidure de N-éthyl-N-méthylpyrrolidinium, le bis-trifluorométhylsulfonylamidure de triéthylsulfonium, le bis-trifluorométhylsulfonylamidure de tributylhexylammonium, le trifluoroacétate de 1-butyl-3-méthylimidazolium et le bis-trifluorométhylsulfonylamidure de 1-butyl-2,3-diméthylimidazolium, seul ou en mélange.

9. Composition catalytique selon l'une des revendications précédentes, dans laquelle l'additif Q₂⁺A₂⁻ est choisi parmi le l'hexafluorophosphate de N-(4-hydroxybutyl)pyridinium, le tétrafluoroborate de N-(2-hydroxyéthyl)pyridinium, l'hexafluoroantimonate de 1-(4-hydroxybutyl)-3-méthylimidazolium, l'hexafluorophosphate de 1-(4-hydroxybutyl)-3-méthylimidazolium, le trifluorométhylsulfonate de 1-(4-hydroxybutyl)-3-méthylimidazolium, le fluorosulfonate de N-(2-hydroxyethyl)pyridinium, l'hexafluorophosphate de triméthyl(2-hydroxyethyl)ammonium, le bis-trifluorométhylsulfonylamidure de 1-(4-hydroxybutyl)-3-méthylimidazolium, le bis-trifluorométhylsulfonylamidure de N-(2-hydroxyethyl)-N-méthylpyrrolidinium, le bis-trifluorométhylsulfonylamidure de diéthyl(2-hydroxyethyl)sulfonium, le bis-trifluorométhylsulfonylamidure de tributyl(4-hydroxybutyl)ammonium, le trifluoroacétate de 1-(4-hydroxybutyl)-3-méthylimidazolium, le bis-trifluorométhylsulfonylamidure de 1-(4-hydroxybutyl)-2,3-diméthylimidazolium et le le bis-trifluorométhylsulfonylamidure de 1-butyl-3-(2-hydroxyéthyl)imidazolium, seul ou en mélange.

10. Composition catalytique selon l'une des revendications précédentes, dans laquelle le rapport molaire entre l'additif Q₂⁺A₂⁻ et le liquide ionique Q₁⁺A₁⁻ est inférieur à 2/1.

11. Composition catalytique selon l'une des revendications précédentes, dans laquelle l'anion B de l'acide de Brønsted est choisi parmi les anions tétrafluoroborate, tétraalkylborates, hexafluorophosphate, hexafluoroantimonate, alkylsulfonates, perfluoroalkylsulfonates, fluorosulfonate, sulfates, phosphates, perfluoroacétates, perfluoroalkylsulfonamides, fluorosulfonamides, perfluoroalkylsulfométhides et carboranes.

12. Composition catalytique selon l'une des revendications précédentes, dans laquelle le rapport molaire de l'acide de Brønsted sur la somme de (Q₁⁺A₁⁻ + Q₂⁺A₂⁻) est inférieur à 2/1.

13. Procédé de dimérisation de l'isobutène mettant en jeu une composition catalytique selon l'une des revendications 1 à 12.

14. Procédé de dimérisation de l'isobutène selon la revendication 13 dans lequel le rapport volumique entre l'isobutène et la composition catalytique est compris entre 0,1/1 et 1000/1.

15. Procédé de dimérisation de l'isobutène selon les revendications 13 et 14 dans lequel on effectue la réaction de dimérisation à une température entre -50°C à 200°C, à une pression allant de la pression autogène à 10 MPa.
